Europäisches Patentamt

European Patent Office

Office européen des brevets

(11) Publication number: **0 214 782 B1**

# EUROPEAN PATENT SPECIFICATION

(45) Date of publication of patent specification: **24.06.92**   (51) Int. Cl.⁵: **G10K 11/34**

(21) Application number: **86306338.4**

(22) Date of filing: **15.08.86**

(54) Ultrasonic irradiation system.

(30) Priority: **16.08.85 JP 179420/85**
**14.10.85 JP 226716/85**
**14.10.85 JP 226717/85**

(43) Date of publication of application:
**18.03.87 Bulletin 87/12**

(45) Publication of the grant of the patent:
**24.06.92 Bulletin 92/26**

(84) Designated Contracting States:
**DE FR GB**

(56) References cited:
**GB-A- 2 113 099**
**GB-A- 2 126 901**
**US-A- 4 324 140**
**US-A- 4 441 486**

**ULTRASOUND IN MED. & BIOL., vol. 8, no. 2, 1982, pages 177-184, Pergamon Press Ltd, GB; R.E. BEARD et al.: "An annular focus ultrasonic lens for local hyperthermia treatment of small tumors"**

**1981 ULTRASONICS SYMPOSIUM PROCEEDINGS, Chicago, 14th-16th October 1981, vol. 2, Editor B.R. McAvoy, pages 632-637, IEEE; M.S. PATTERSON et al.: "Sidelobe and speckle reduction for an eight sector conical scanner"**

(73) Proprietor: **HITACHI, LTD.**
**6, Kanda Surugadai 4-chome**
**Chiyoda-ku, Tokyo 100(JP)**

(72) Inventor: **Umemura, Shinichiro**
**C403 Hitachi Koayasu Apt. 2-32, Koyasu-cho**
**Hachioji-shi Tokyo(JP)**
Inventor: **Cain, Charles Arain**
**507, West Pennsylvania**
**Urbana Illinois 61801(US)**
Inventor: **Katakura, Kageyoshi**
**1-25-32, Naka-cho Meguro-ku**
**Tokyo(JP)**

(74) Representative: **Calderbank, Thomas Roger et al**
**MEWBURN ELLIS & CO. 2/3 Cursitor Street**
**London EC4A 1BO(GB)**

## Description

The present invention relates to a local hyperthermia ultrasonic irradiation system with annular focal zone suitable for remedy of malignant tumours.

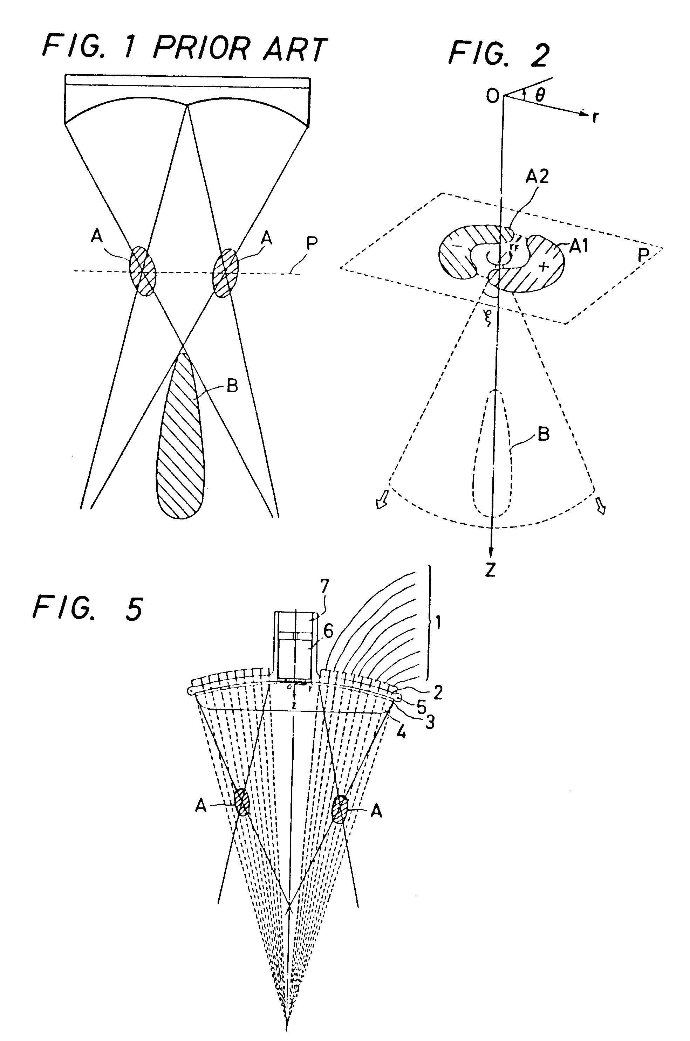

In such systems involving medical treatment, the ultrasound focal zone formed by a single spot focus may be too small, as compared with the target zone to be irradiated. To overcome this, it is known to provide an ultrasonic irradiation system in which an annular focal zone is formed by using an acoustic lens such as that disclosed in "Ultrasound in Med. & Biol." vol. 8, No. 2 (issued in 1982), pp. 177 to 184. However, this system has the defect that the position, size and shape of the irradiation cannot be controlled in dependence on the object to be irradiated with ultrasonic waves because they are fixed. Moreover, the system has a tendency that the ultrasonic waves spreading again from an annular focal zone A will reform a long column-shaped focal zone B on the centre axis of the annulus, as shown in section in Fig. 1. This tendency is sufficiently acute to be a serious problem when the diameter of the annulus is smaller than that of the ultrasonic probe. In the field of ultrasound therapy, for example, the formation of a secondary focal zone, in addition to the target zone, may adversely affect the normal internal tissues of a patient or may cause pain.

In the article "Sidelobe and Speckle Reduction for an Eight Sector Conical Scanner" by Paterson et al, 1981 Ultrasonics Symposium proceeding, Chicago, 14-16 October 1981, vol. 2, pages 632-63, IEEE, an ultrasonic irradiation system is disclosed having a transducer being divided into a plurality of elements, a driving circuit for driving each of the elements respectively, so that the system can be used for antifocus processing of the signals on the sound pressure regions.

GB-A-2126901 discloses an ultrasonic hyperthermia system comprising a plurality of transducers for transmitting coherent beams, so that the beams can be focused for uniformly heating a limited part of the human body.

US-A-4,441,486 discloses another ultrasonic hyperthermia system comprising a plurality of transducers mounted in an isospherical configuration so that the corresponding beams are directed toward a central axis of the system for a concentrated heating of a region.

The present invention seeks to provide a local hyperthermia ultrasonic irradiation system capable of forming a larger annular focal zone than a single spot focus which may also permit the area of that zone to be variable. It is also desired to form a larger focal zone than the single one without forming a secondary focal zone, in addition to the target zone.

According to a first aspect of the present invention, there is provided a local hyperthermia ultrasonic irradiation system with annular focal zone comprising:

a transducer having a generally circular shape and being divided into a plurality of elements at least in a circumferential direction, the transducer being adapted to generate a plurality of sound pressure regions on a focal plane;

a drive circuit for generating drive signals for driving each of the elements, respectively, wherein the system has a control circuit for controlling the phases of the drive signals such that the modulus or root mean square value of the integral over the focal plane of the sound pressures of the sound pressure regions is negligible compared with the integral of the modulus or root mean square value of the sound pressures over the focal plane.

According to a second aspect of the invention there is provided a local hyperthermia ultrasonic irradiation system with annular focal zone comprising:

a transducer having a generally circular contour and being divided into a plurality of elements in both circumferential and radial directions;

a drive circuit for driving each of the elements respectively, wherein the system has a control circuit for controlling the phases of drive signals for driving the elements, respectively, wherein the control circuit is adapted to control the phases of the respective drive signals for driving the transducer elements such that sound waves may be converged in a desired annular focal zone and such that the modulus of the integral of the sound pressure over the focal plane is negligible compared with the integral of the modulus of the sound pressure over the same plane.

According to a third aspect of the present invention, there is provided a local hyperthermia ultrasonic irradiation system with annular focal zone comprising:

a transducer having a plurality of elements arranged in a two-dimensional array;

a drive circuit for driving the elements respectively, wherein the system has a control circuit for controlling the phases of drive signals for driving the elements, respectively, wherein the control circuit is adapted to determine the term of a phase adjustment corresponding to the respective radial positions of transducer elements such that the integral of the sound pressure over the focal plane is negligible compared with the integral of the modulus of the sound pressure over the same plane, and such that sound waves are convergeable at the centre position of the focal plane of a desired annular focal zone, and is adapted to reverse sequentially the polarities of the drive signals in a predetermined direction of the two-dimensional array.

According to a further aspect of the present invention, there is provided a local hyperthermia ultrasonic irradiation system with annular focal zone comprising:

a transducer having a plurality of elements arranged in an annular array;

a drive circuit for driving the elements respectively, wherein the system has a control circuit for controlling the phases of drive signals for driving said elements, respectively, wherein the control circuit controls the phases of the respective drive signals of the transducer elements such that the integral of the sound pressure over the focal plane is negligible compared with the integral of the modulus of the sound pressure over the same plane, and such that sound waves are convergeable toward an annular focal zone having a desired radius and a desired depthwise position.

The adjustment of the phases of the drive signals in the radial direction is unnecessary if it is sufficient to fix the focal distance. Therefore, this focal plane can be formed either by using an acoustic lens or by making the transducer face concave. In this case, the transducer may be of radial array type in which a transducer plate of a circular or a similar shape is divided into a plurality of elements along the circle.

In order to control the size of the focal zone, on the other hand, the number of poles zn (n = 1, 2, 3, 4 and so on) of the drive signals in circumferential direction of the transducer face is changed. The diameter of the annular focal zone is enlarged by increasing the polar number n.

Embodiments of the invention will now be described in detail, by way of example, with reference to the accompanying drawings, in which:

Fig. 1 is a sectional view showing the probe and its focal zone produced by a known ultrasonic irradiation system, and has already been described;

Fig. 2 is a diagram showing the focal zone produced by an embodiment of the present invention;

Fig. 3 is a block diagram showing one embodiment of the present invention;

Figs. 4A and 4B, Figs. 6A and 6B, and Figs. 8A and 8B are respectively top and sectional views showing examples of a probe to be used in the present invention;

Fig. 5 is a diagram showing the focal zone formed by the probe of Figs. 4A and 4B;

Fig. 7, Figs. 9A and 9B, and Figs. 10A, 10B and 10C are respectively top views showing phase controls for the probes;

Figs. 11A, 11B and 11C are diagrams obtained by the phase controls of Figs. 10A, 10B and 10C;

Figs. 12A and 12B are a top and a sectional view respectively of a probe for use in another embodiment of the present invention;

Figs. 13A and 13B are diagrams showing sound waves obtained by the probe of Figs. 12A and 12B; and Figs. 14A and 14B are a top and a sectional view respectively showing a probe for use in a further embodiment of the present invention.

Fig. 2 shows the pattern of a sound field formed by a representative embodiment of the present invention.

If the phase of the sound pressure of an annular focal zone on a focal plane P is deliberately modulated along the annulus so that the integration of sound pressure on P is substantially equal to zero, the acoustic energy necessary for forming annular focal zones A1 and A2 on the focal plane P can be concentrated without forming any secondary focal point B. In other words, the focal zones formed on the focal plane P are symmetric with respect to the centre axis so that the sound pressure on the axis is maintained at zero.

If, moreover, the point where the phase of the sound pressure is zero is rotated, as indicated by thick arrows in Fig. 2, the acoustic energy time-averaged on the annulus may be made uniform, to form the desired acoustic energy irradiation pattern.

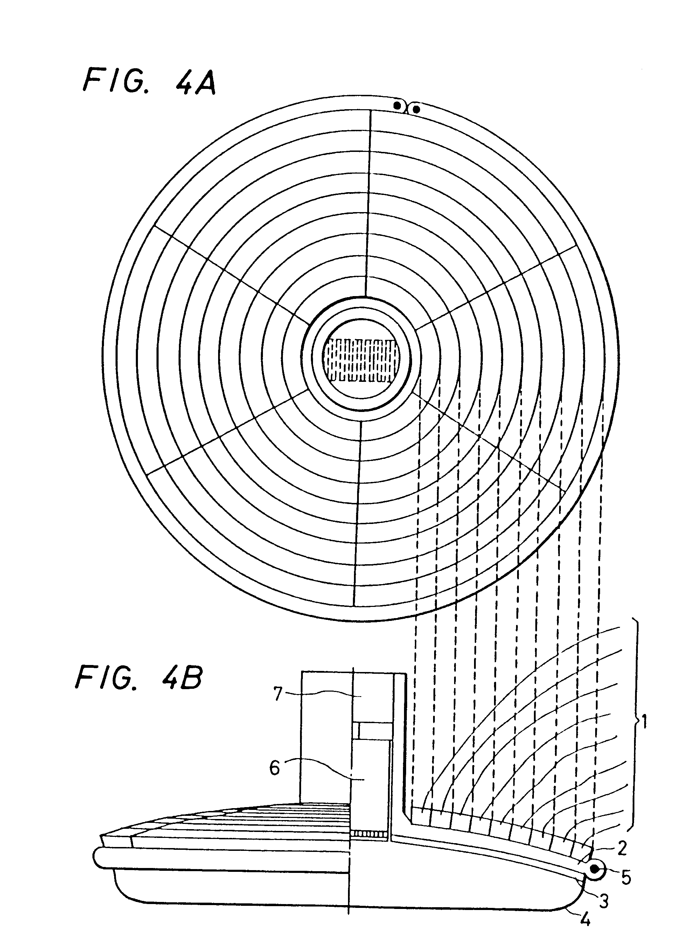

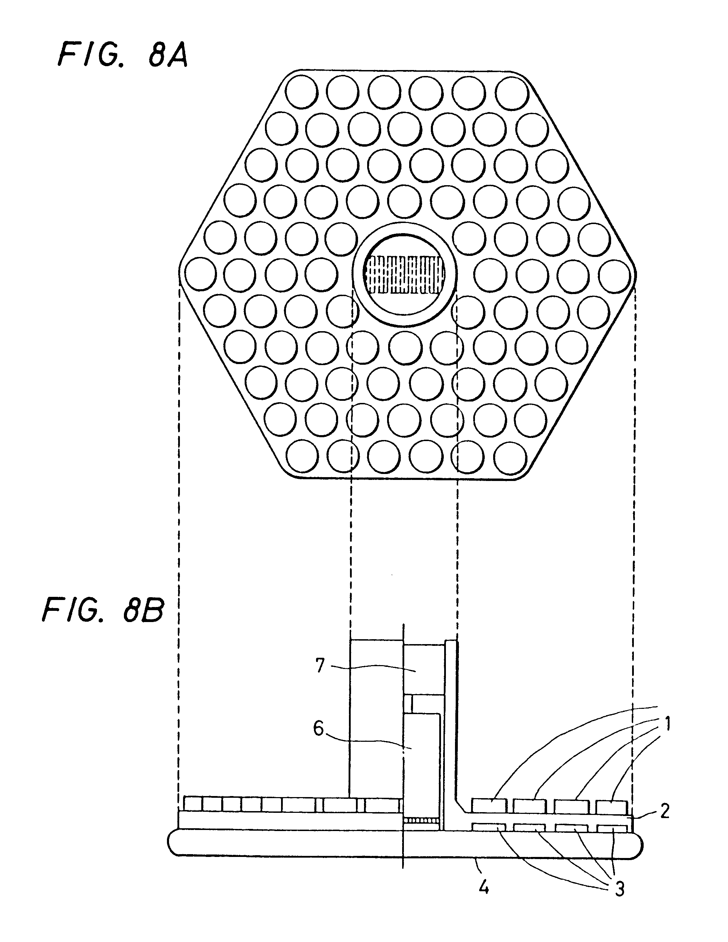

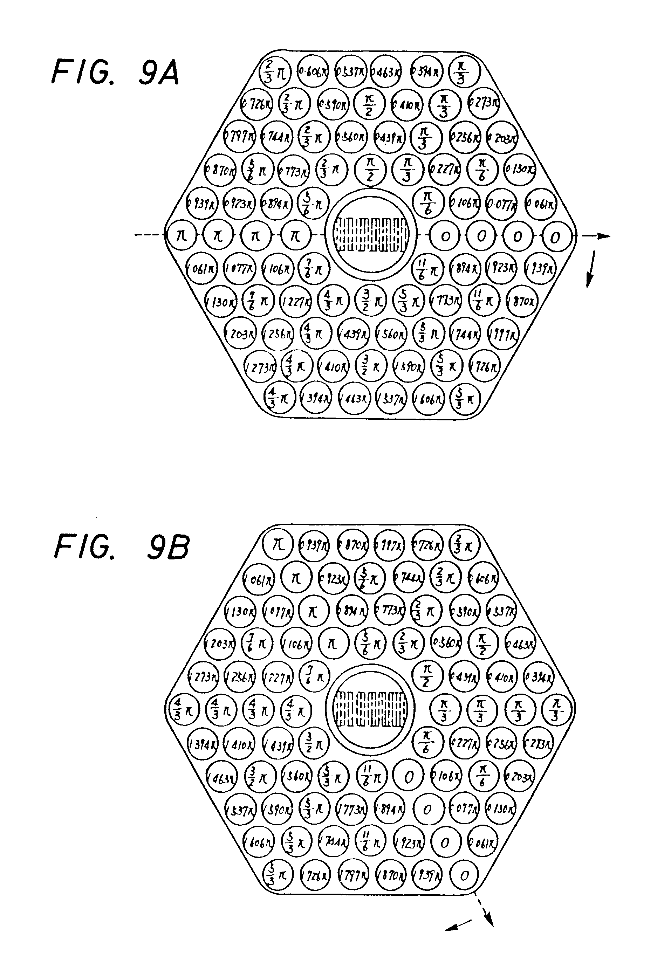

The constructions of the probes shown in Figs. 4A and 4B, Figs. 6A and 6B, and Figs. 8A and 8B will now be described in detail. In all these examples of a probe suitable for use in the present invention, a transducer 1 made of piezoelectric ceramics and a second acoustic matching layer 3 made of a polymer are adhered to the front and back of a board 2 of light metal acting as a first acoustic matching layer, as a ground electrode and as a heat sink, respectively. A water bag 4 for acoustic coupling between the transducer and an object to be irradiated is attached to the second acoustic matching layer 3. In the probe of Figs. 4 and 6, the transducer 1 is divided into ring-shaped oscillating elements, each of which is radially divided into six oscillating elements. In the probe of Fig. 8, on the other hand, the oscillator is constructed by arraying a plurality of oscillating elements regularly two-dimensionally. Thus, the oscillator for irradiating sound waves to an annular focal zone is constructed of those plurality of oscillating elements. In all the examples of Figs. 4, 6 and 8, an auxiliary probe 6 for monitoring the irradiation is rotatably fitted in the central portion of the probe. That auxiliary probe 6 is of a small linear array type, in which the transducer elements have a resonance frequency between 100 kHz and 10 Mkz, which is selected to be two or more times higher than the resonance frequency of the transducer 1. In a suitable example, the transducer 1 has a resonance frequency of 500 kHz whereas the auxiliary probe 6 has a resonance frequency of 3 MHz. The orientation of the array of the auxiliary probe 6 is controlled by means of a motor 7.

Moreover, a cooling pipe 5 is provided in the light metal board 2 of Figs. 4 and 6. In the example of Fig. 8, however, there is no need for a cooling pipe because the light metal board 2 directly contacts the acoustic coupling water confined in the water bag 4.

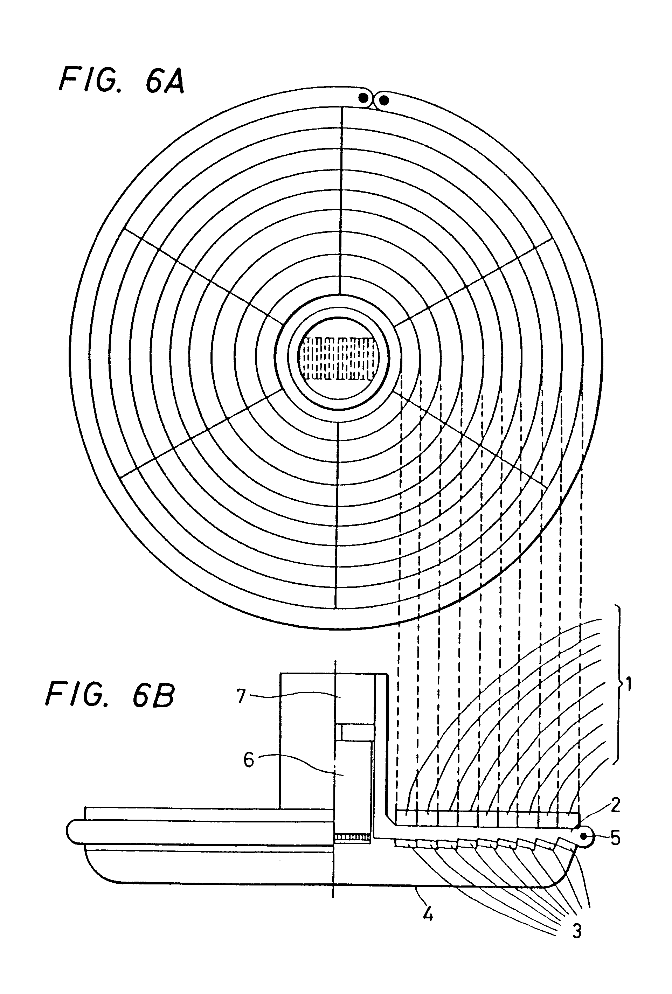

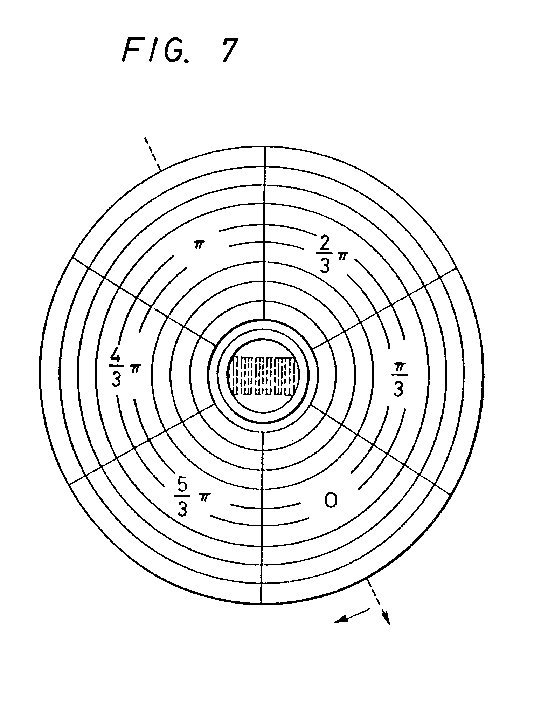

In the embodiment of Fig. 4, as shown in section in Fig. 5, the probe is given a finite curavature R so as to minimize the number of the transducer elements necessary for scanning the target zone by changing the radius and depth of the annular focal zone A. By thus setting the maximum direction of the directivity of the outer elements inward, the number of the elements required can be reduced to about one half the number needed with a planar probe. A similar effect can also be attained by combining a planar oscillator and an acoustic lens. This combination is shown in the example of Fig. 6, in which the acoustic lens is prepared by machining the light metal board 2 to form a Fresnel lens.

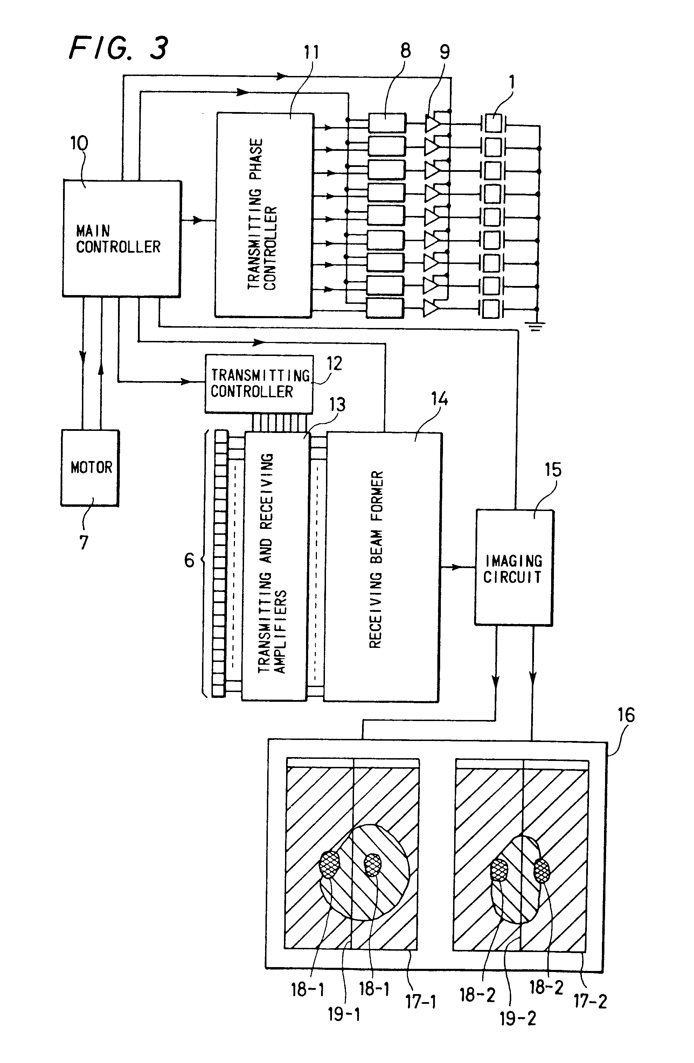

The overall construction of the system will be described with reference to Fig. 3. A main controller 10 supplies a signal to a transmitting phase controller 11, the signal determining the radius of an annular focal zone to be formed, the distance from the probe and the modulation mode along the annulus. As will be described later in detail, the drive phases of the respective elements of the transducer 1 in the transmitting phase controller 11 are determined by the signal from the main controller 10 until they are transferred as load data to m-bit counters 8. These m-bit counters 8 operate using those load data as their initial values, and they count clock pulses of a frequency of $2^m \cdot f_0$ from the main controller 10. The m-bit counters 8 outputs their highest-bit signals, to produce irradiating transmission phase signals of a frequency $f_0$ which are fed to transmission amplifiers 9. These transmission amplifiers 9 amplify those transmitting phase signals to amplitudes determined by the signal from the main controller 10, and the amplified signals drive the respective elements of the transducer 1. As a result, the drive phases of the respective elements are individually controlled.

An example of the drive phases necessary for generating the sound field of the type shown in Fig. 2 will now be described. It is assumed here, as shown in Fig. 2; that the axis of rotational symmetry of the probe is the Z-axis; that cylindrical coordinates using the intersection between the probe and the Z-axis as an origin are designated at $(Z, r, \theta)$; that the coordinates of the centre of a k-th transducer element are designated at $(Z_k, r_k, \theta_k)$; and that the coordinates of the annulus of the annular focal zone are designated by $Z = Z_F$ and $r = r_F$. With these assumptions, the drive phase $\phi_k$ (wherein k designates a natural number) to be given to each element is generally expressed by the following equation:

$$\zeta_k(Z_k, r_k, \Theta_k, t) = \alpha(Z_k, r_k) + \beta(\Theta_k) + \omega_0 \cdot t \quad (3),$$

wherein $\omega_0$ designates the angular frequency of the ultrasound waves to be transmitted.

In the right-hand side of the equation for determining the drive phase, the function $\alpha$ of $(Z_k, r_k)$ provides factors for determining the depth $Z_F$ of the focal plane and the radius $r_F$ of the annular focal zone. Two methods exist for calculating the function $\alpha$. One method is to determine the drive phase of each element such that the sound waves may converge at the position $(Z_F, r_F)$ of the annulus in the section of the probe. According to this method, the function $\alpha$ is given by the following equation:

$$\alpha(Z_k, r_k) = 2\pi/\lambda_0 [\sqrt{(Z_k - Z_F)^2 + (r_k - r_F)^2} - Z_F] \quad (4)$$

Another method is to form the annular focal zone by modulating the driving phases, which are calculated to locate the focal point at $(Z_F, 0)$, by a further modulation by changing the drive polarity of each element. In order to consider what drive polarity is to be given, it is convenient to invert the time axis in the propagation of the sound waves. Since the sound wave A by the annular sound source having a radius $r_F$ is expressed by the following equation:

4

$$A \propto J_0 \left( \frac{2\pi r_F}{\lambda_0} \sin\theta \right) \doteq J_0 \left( \frac{2\pi r_F}{\lambda_0}\theta \right) \quad \cdots(5)$$

(wherein: $J_0$ designates the 0-th order Bessel's function; and $\theta$ designates an azimuth angle). The sound field is generally proportional to $J_0$ $(2\pi$ $r_F/\lambda_0 . r_k/Z_F)$ on a circumference of a radius $r_k$ on the face of the probe spaced at a distance $Z_F$ from the focal plane. Therefore, the annular focal zone of the radius $r_F$ from the focal plane. Therefore, the annular focal zone of the radius $r_F$ at the distance $Z_F$ from the probe can be formed by giving the following drive phase to each element. If the h-th zero point of the 0-th order Bessel's function is designated at $a_h$ and if the following equation holds:

$$r_c = \frac{\lambda_0 Z_F}{2\pi r_F} \quad \cdots(6)$$

i) $r_k < a_1 r_C$ or $a_{2h} r_C < r_k < a_{2h+1} r_C$:
$\alpha(Z_k, r_k) = \frac{2\pi n}{\lambda_{t,>}} [\sqrt{(Z_k - Z_F)^2 + r_k^2} - Z_F]$ (7)
ii) $a_{2h-1} r_C < r_k < a_{2h} r_C$:
$\alpha(Z_k, r_k) = \frac{2\pi n}{\lambda_{t,>}} [\sqrt{(Z_k - Z_F)^2 + r_k^2} - Z_F] +$ (8)

(wherein h designates a natural number).

I the probe has a constant radius o curvature R, as shown in Fig. 4, the following equation holds in the equations (4), (7) and (8):

$Z_k^2 - 2RZ_k + r_k^2 = 0$ (9)

If on the other hand, the probe has an infnite radius o curvature, as shown in Fig. 8, the following equation holds:

$Z_k = 0$ (10)

With either method, the depth $Z_F$ o the focal plane and the radius $r_F$ o the annular focal zone are determined by the term $\alpha$ o the phase in dependence on the radial position $r_k$ o the oscillating elements.

On the righthand side o the equation determining the drive phase, the term $\beta$ o the function o $\Theta_k$ is calculated in the following manner. In the case o the dipolar annular focal zone o Fig. 2, the function $\beta$ is given by the following equation:

$\beta(\theta_k) = \theta_k$ (11)

In other words, the phase o the drive signal o each element is rotated 360 degrees for one rotation in the circumferential direction on the face o the probe. In the probe o Fig. 4 or 6, the distribution o the phase o each element has the shape shown in Fig. 7. The distribution for the embodiment o Fig. 8, however, has the shape shown in Fig. 9. In this embodiment, the phase distribution is rotated whilst the phase difference between the elements is maintained. Since the equi-phase plane o the sound pressure is rotated in the direction o the arrow, as shown in Fig. 2, on the focal plane P, that rotation o the phase distribution causes the energy o the sound waves to be distributed uniformly in an annular form as a time elapses.

Although the above description has been concerned with the case in which a dipiar focal zone is formed, the polar number can be increased to form a focal zone o 2n poles. In this case, the term $\beta(\theta_k)$ o the equation (3) is given by the following equation:

$\beta(\theta_k) = n\theta_k$ (12)

Thus the drive phase $\phi_k$ obtained fom equation (3) is quantitized in units o $2/2^m$, so that the lower m bits are outputted fom the transmitting phase controller 11 to produce the load data o the m-bit counters 8.

As a result, each element is driven by the phase which is designated by $\phi_k$.

In the example o Fig. 7, the number o driving amplifers 9 in Fig. 3 can be decreased by hal by changing the piezoelectric polarity o transducer elements 1. For instance, positive polarity is given to '0', '/3', and '2/3' elements in Fig. 7, negative polarity is given to others, and point symmetric pairs o elements are electrically combined by pairs.

The embodiment o Fig. 3 is equipped with an irradiation monitoring imaging means in addition to the irradiating transmitting phase means, as will now be described. In Fig. 3, there is shown an imaging auxiliary probe 6, and a motor 7 or rotating the probe 6 on the Z-axis so that a plurality o ultrasound echo tomograms necessary or positioning the irradiation target can be ormed. Each o the elements o the auxiliary probe 6 is connected, through a transmitting and receiving amplifer 13, to a transmitting controller 12 and a receiving beam ormer 14. The drive signals, which have their phases controlled or each element by the transmitting controller 12, are repeatedly applied through the transmitting and receiving amplifers 13. As a result, an ultrasound beam or imaging by linear or sector scanning is repeatedly emitted fom the probe 6 into a predetermined part o the object. This ultrasound beam has a fequency equal to the resonance fequency o the auxiliary probe 6.

Both the echo signals generated due to discontinuity o the acoustic impedance in the object and the harmonics signals generated due to the acoustic nonlinear eóct by the irradiating ultrasound waves are received by the respective elements o the imaging auxiliary probe 6, amplifed by the transmitting and receiving amplifers 13, and ócused by the receiving beam ormer 14. As a result, signals are generated which are indicative o the time changes in the intensity o the refected signals or the harmonics signals based on the received beam which is sequentially scanned or imaging by linear or sector scanning. These signals are ód through an imaging circuit 15 to a display unit 16 so that the generated positions and the ultrasound intensities o the echo signals or the harmonics signals are displayed through the imaging circuit 15 in the display fame o the display unit 16. The receiving beam ormer 14 is equipped with electronic scanning means, electronic ócusing means, and also a band-pass flter so that its centre fequency is synchronised with the imaging ultrasound fequency, which is more than twice as high as the irradiating ultrasound fequency. This makes the ultrasonic imaging operation possibile without any interórence, even during ultrasonic irradiation. The display fame stores and displays two images, as designated at 17-1 and 17-2, so as to make it convenient to position the irradiation target. Therefóre, the operator operates the auxiliary probe by means o the motor 7 so that the tomograms taken in two arbitrary directions can be monitored by means o the display unit 16. Moreover, the monitor unit o the present embodiment superposes the tomographs o the object to display the ócal zone o the ultrasound beam emitted fom the oscillator 1 in the órm o markers (as designated at 18-1 and 18-2 in Fig. 3). The imaging circuit 15 generates the marker signals indicating the section o the ócal zone by using the signals indicating the depth and radius o the annular ócal zone fom the main controller 10 and óeds them to the display unit 16. Markers 19-1 and 19-2 indicate the positions o the centre axis o the probe.

By this imaging means, the óllowing kinds o irradiation monitoring operations may be accomplished:

(1) The irradiation target may be identifed and positioned by the ultrasonic imaging;

(2) The movement o the irradiation target may be detected, so that the irradiation zone may be moved in accordance with the target movement detected;

(3) The change in the acoustic impedance and in the sound velocity due to the temperature rise o the irradiation zone may be observed by measuring the intensity and the echo signal refected fom the irradiation zone;

(4) The harmonics waves generated in the irradiation zone by acoustical non-linear eócts may be observed; and

(5) The harmonics waves generated in a so-called "hot spot" other than the target zone may be monitored.

Operation 1 has the advantage that it is not signifcantly infuenced by refaction, even i sound velocity has a distribution in the human body, because the target is positioned by ultrasound waves which are similar to the irradiated waves and not by other means such as X-rays. Operation 2 makes use o the high speed o the ultrasound pulse echo imaging and is particularly suitable ór electronic scanning type ultrasonic imaging means. Since movement o the irradiation target in three dimensions has to be detected, three-dimensional scanning with the ultrasound beam is necessary using a two-dimensional array type probe or by combining electronic scanning and mechanical scanning. Operation 3 observes changes in the refected echo intensity, which is caused by changes in the acoustic impedance or the product o the sound velocity and the specifc gravity both changing. Such changes result when the temperature o a substance in the irradiated zone is raised by absorption o the ultrasound wave. Operation 3 also detects changes in the refected echo phase, which are caused by changes in sound velocity.

Thus, operation 3 is particularly eíective in monitoring ultrasound heating. This method is partly disclosed on p. i.788 o "American Acoustics Report", vol. 15, No. 11 and in US-A-4,566,459.

The non-linear acoustic parameter A/B o a substance or cavitation in the irradiation zone causes the generation o harmonics components o the irradiating ultrasound waves. These are observed by operation 4 to provide iníormation concerning the intensity and action o the ultrasound waves in the generation zone. I the waves having a relatively high reíection intensity are present in the irradiation target medium o the ultrasound waves, standing waves may be produced in the irradiation target medium to cause a so-called "hot spot" in parts o the medium other than the irradiation target zone. This unintended irradiation zone may be dangerous, particularly when the present invention is used in medical treatment so that it has to be avoided by careíul monitoring.

Operation 5 monitors hot spots by observing the harmonics waves which are generated by the non-linear acoustic eííct.

In order to carry out operations 4 and 5, it is necessary to stop the operation o the transmitting controller 12 thereby to detect only the harmonics waves which are geneated as a result o the irradiation o the sound waves using the probe 1. On the other hand, i a colour display unit is used as the display unit 16 and i the imaging circuit 15 generates display signals o diíerent colours when the amplitude o the harmonics waves detected exceeds a predetermined allowable limit, this is more preíerable as it immediately attracts the attention o the operator.

In the embodiment thus íar described, the depth $Z_F$ and radius $r_F$ o the annular íocal zone are determined by the phase adjustment (i.e. the term o $\alpha(Z_k, r_k)$ o equation (3)) according to the radial position $r_k$ o the drive signal o the divided transducer 1. However, the radius $r_F$ can also be controlled by the polar number (i.e. zn o equation (12)) o the rotations o the drive signals along the circumíerential position o the oscillator. Thereíore, the term o $\alpha(Z_k, r_k)$ o the equation (3) may be determined by the íollowing equation, rather than by equation (4):

$$\alpha(Z_k, r_k)$$
$$= 2/\lambda_0 (\sqrt{(Z_k - Z_F)^2 + r_k^2} - Z_F) \qquad (13)$$

Equation (13) implies that the drive phase determined by the radial position $r_k$ o the elements o the probe 1 may be calculated so that it is íocused in the centre position o the íocal plane at the desired depth $Z_F$.

When the probe o Fig. 8 is used, on the other hand, the drive phase may be inverted into a stripe íorm at the position o the two-dimensional array, rather than adjusting the drive phase according to the element position o the probe in the circumíerential direction, as expressed by the equation (11). Then, the drive phase $\phi_k$ o the probe element located at $(Z_k, r_k, \theta_k)$ is expressed by the íollowing equation:

$$\phi_k(Z_k, r_k, \theta_k) = \tfrac{2\pi n}{\lambda t,>} [\sqrt{(Z_k - Z_F)^2 + r_k^2} - Z_F]$$
$$+ \gamma(Z_k, r_k, \theta_k) + \omega_0 t \qquad (13)$$

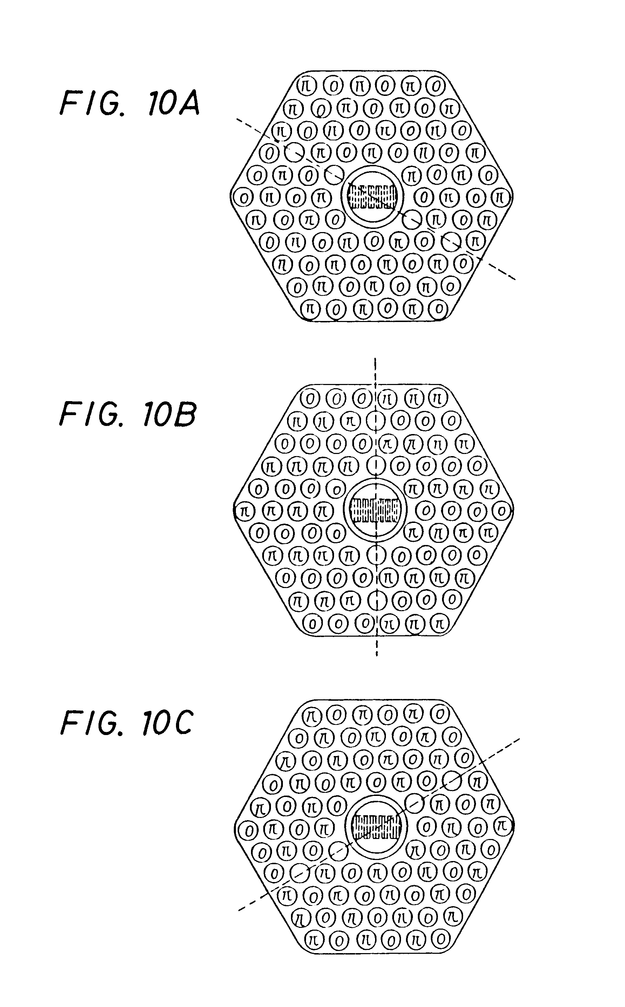

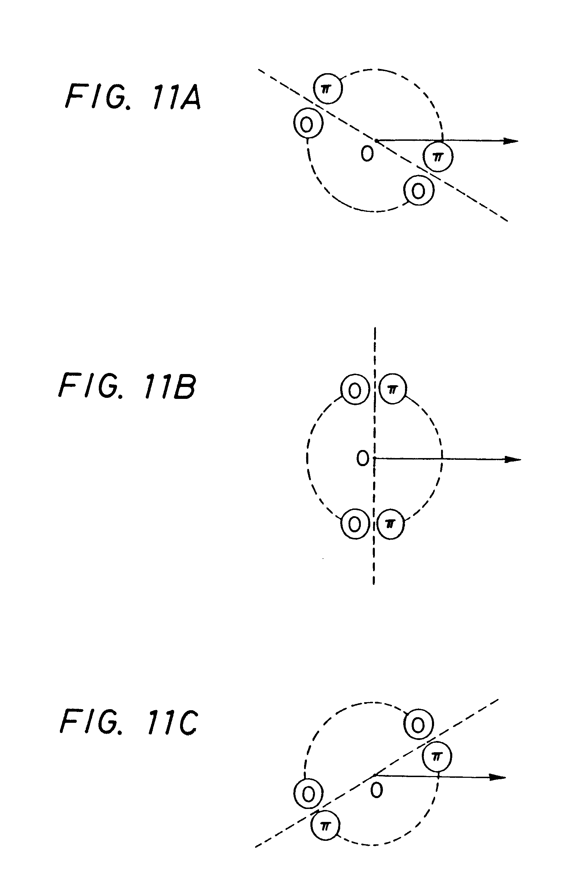

Here, the phase denoted by $\gamma$ is shown in Figs. 10A, 10B and 10C, íor example. By thus controlling the drive phase, a plurality o spots o diíerent polarities can be íormed simultaneously on the íocal plane P, as shown in Figs. 11A, 11B and 11C. I irradiation is carried out whilst simultaneously switching the modes o Figs. 11A, 11B and 11C, the ultrasound wave energy can also have an annular time averaged distribution. By using a speciíed one o the modes o Figs. 11A, 11B and 11C, moreover, it is possible to íorm an ultrasound wave energy distribution which is suitable íor a rotationally asymmetric irradiation target zone.

One íeature that the examples o Figs. 7, 9 and 10, which show the distributions o the phase o the sound pressures on the íaces o the transducer elements o a two-dimensional array type ultrasound transducer, have in common is that the drive phases o the respective elements are controlled so that the integral o their value is substantially negligible compared with the integral o their absolute values. This control provides an eíective method íor íorming a sound íeld in which the integral o the sound pressure can be substantially neglected in comparison with the integral o the absolute value o the sound pressure on the íocal plane.

As a result, the annular íocal zone can be íormed without íorming the secondary íocal zone B shown in Fig. 1.

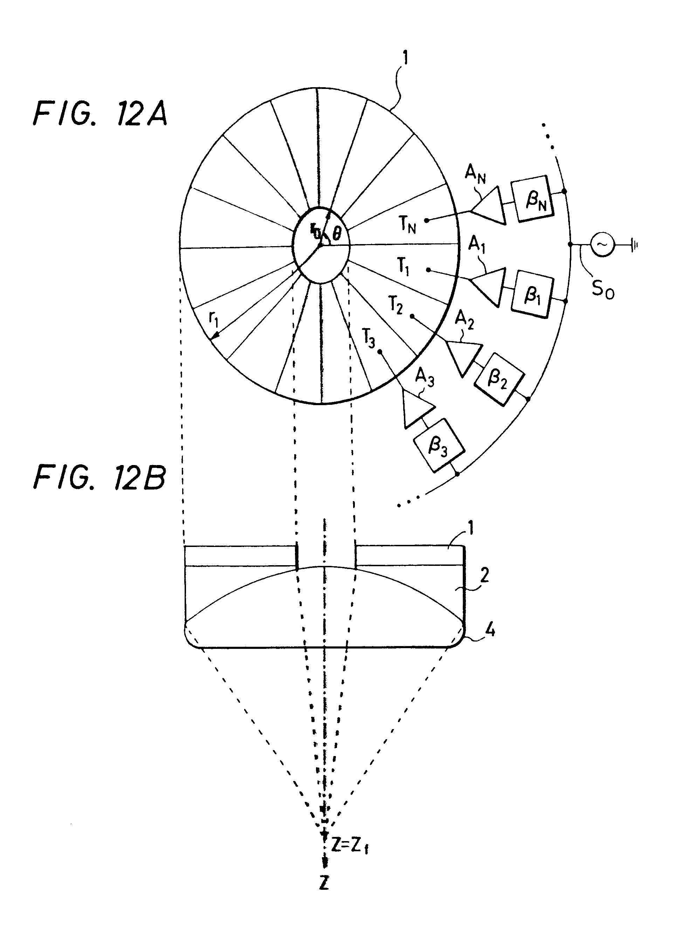

Figs. 12A and 12B show the structure o a probe íor use in a system being a íurther embodiment o the present invention. This probe has a íxed íocal point using an acoustic lens to replace the control o the drive phase using the term $\beta(Z_k, r_k)$ o equation 3 and íorms an annular íocal zone. The transducer 1 íor the sound wave transmission is divided into a plurality o annular piezo-electric elements $T_1$, $T_2$, - - -, and $T_N$ having an internal radius $r_0$ and an external radius $r_1$. These elements are attached to the back o an acoustic lens 2 having a íocal length $Z_F$. Also shown in Fig. 12B is a water bag 4.

I the transducer elements are considered in polar coordinates (r, $\theta$), the angular coordinate o the i-th element are designated at $\theta_i$ and the amplitude o the drive signal is designated at $A(\theta_k)$, then the drive signal is controlled to satisy the ollowing equation:

$$A(\theta_k) = A_0 e^{j[n(\theta_k + \beta_i(\theta_k)) - \omega_0 t]} \quad - - - - - (14).$$

Thus the control is such that the phase o the drive signal proceeds on the annular transducer in the circumrential direction at an angular velocity $\omega_p$ given by the ollowing equation:

$$\omega_p = \omega_0 / n \cdot (1 + \beta_i'(\theta_k)) \quad - - - - - - (15)$$

Here: $\omega_0$ designates the angular velocity o the ultrasound waves; n designates the number o phase rotation per rotation in the circumrential direction $\beta_1'(\theta_k)$ designates a unction expressing the azimuth modulation o the phase angular velocity; and $A_0$ designates a constant.

For simplicity, the sound feld B on the ocal plane in the absence o the modulation $\beta_1'(\theta_k)$ will now be calculated. I the polar coordinates on the ocal plane are designated at $(R, \textcircled{H})$, i the wave number o the ultrasound waves is designated at k, and i the ollowing equation holds:

$$K_F = kR/Z_F \quad (16),$$

then the ollowing equation is obtained:

$$B = \int_{r_0}^{r_1} \int_0^{2\pi} A(\theta) e^{jK_F r \cos(\theta - \textcircled{H})} \, r d\theta dr$$

$$= A_0 e^{j[n(\textcircled{H} + \pi/2) - \omega t]} \int_{r_0}^{r_1} \int_0^{2\pi} e^{j(n\theta - K_F r \sin\theta)} \, d\theta dr$$

$$= A_0 e^{j[n(\textcircled{H} + \pi/2) - \omega t]} \int_{r_0}^{r_1} J_n(K_F r) 2\pi r d\theta \quad - - - (17).$$

I $r_2 = 2/3 \cdot (r_1^3 - r_0^3) / (r_1^2 - r_0^2)$, the equation (17) can be approximated into the ollowing orm:

$$B = A_0 e^{j[n\textcircled{H} + \pi/2) - \omega t]} \cdot \pi(r_1^2 - r_0^2) J_n(k_F r_2)$$

$$- - - (18).$$

In other words, the sound feld B has a radial distribution with the orm o an n-th order Bessel's unction. As a result, annular ocal zones o dierent radii can be ormed by changing the phase rotation number n.

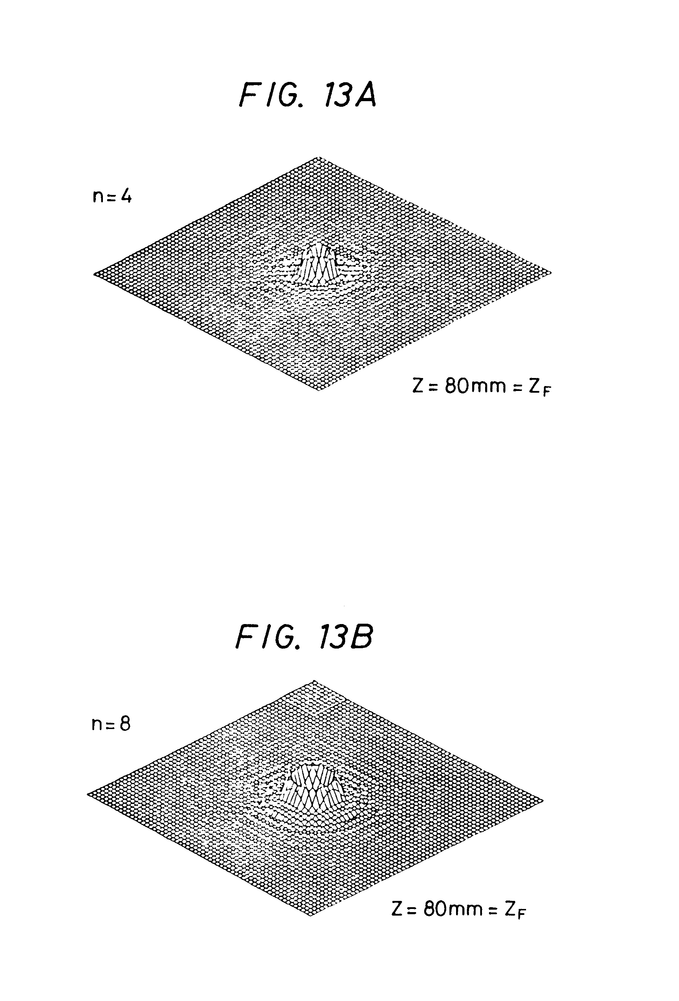

Figs. 13A and 13B show the sound pressure distribution (in absolute values) o the sound feld on a ocal plane, which is established or an ultrasound fequency o 0.5 MHz, a transducer having an internal radius o $r_0$ = 20 mm and an external radius $r_1$ = 60 mm, a ocal length o $Z_F$ = 80 mm and a probe having an element number N = 64, and when the phase rotation number n o the drive signal per rotation is set at n = 4 and n = 8. It is ound that the radius o the ocal zone on the ocal plane is substantially proportional to the phase rotation number n.

Furthermore, an elongated annular focal zone is formed by modulating the angular velocity $\omega_p$ given by equation (15) using the modulation term $\beta_1(\theta_k)$. For example, when the transducer in Fig. 12A and 12B is driven by the signal with n = 8 and

$$\beta_1(\theta_i) = 0.15 \sin 2\theta, \qquad (19)$$

an oval-shaped focal zone with an aspect ratio 1.35 is formed on the focal plane.

The arrangement of Fig. 13 shows the use of the acoustic lens for geometrical focusing (i.e. to determine the focal length $Z_F$). However, geometrical focusing can also be achieved by making the shape of the transducer elements concave.

In the arrangements discussed above, the shapes of the transducers were circular or formed a regular polygon. This invention can also be applied to the transducers with other types of shapes such as elongated circles or elongate polygons. Also, these arrangements form the annular focal zone and control its diameter by using a probe having a two-dimensional or radially divided array of elements and by rotating the phases of the driving signals in the circumferential direction. However, the formation of the annular focal zone and the control of the zone radius can also be achieved by using an (annular array) probe composed of a number of multi-ring-shaped transducer elements.

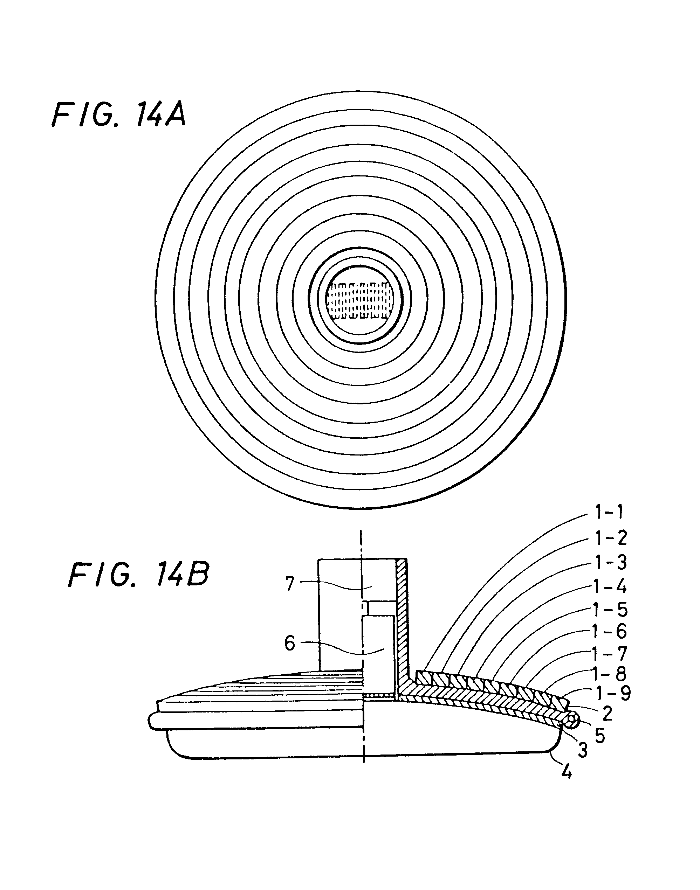

Fig. 14 shows this arrangement, in which reference numerals 1-1, 1-2, 1-3, - - -, and 1-9 designate transducer elements divided into a multiplicity of rings, with the parts being indicated by the same numerals as those of the arrangement of Fig. 4. If cylindrical coordinates are considered with the rotationally symmetric axis of the probe located on the Z axis and an origin located at the centre of the probe and if the coordinates of a k-th transducer element are $Z = Z_k$ and $r = r_k$ and the coordinates of the annulus of the central portion of an annular focal zone are $Z = Z_F$ and $r = r_F$, the phase $\phi_k$ of the drive signal to be fed to that k-th transducer element is given by the following equation:

$$\phi_k = \frac{2\pi n}{\lambda_{1,>}} \left[ \sqrt{(Z_k - Z_F)^2 + (r_k - r_F)^2} - Z_F \right] \qquad (20)$$

Here, $\lambda_0$ designates the wavelength of the irradiation ultrasound waves.

Thus, in conclusion, the present invention proposes that the system has a control circuit for controlling the phases of the drive signals such that the modulus or root mean square value of the integral over the focal plane of the sound pressures of the sound pressure regions is negligible compared with the integral of the modulus or root mean square value of the sound pressures over the focal plane.

When the field pattern is static, this has the effect that the value of the integral over the focal plane of the sound pressures of the sound pressure regions generated by the transducer is negligible compared with the integral over each sound pressure region of the sound pressure of that sound pressure region at any time. When the field pattern is not static however, for example when the transducer illustrated in Fig. 12 A and B is driven with the signal in equation (14), the field pattern rotates in the order of magnitude of the ultrasound frequency. Thus the sound pressure $P^*$ on the focal plane S is controlled to satisfy the following inequality.

$$\left| \iint_S P^* \, dxdy \right| \ll \iint_S |P^*| \, dxdy \qquad (1)$$

where

$$P^* = P_0(x,y)e^{jwt} \qquad (2)$$

$$P^* = P_0(x,y) \qquad (3)$$

Alternatively, the following expression could be used:

$$\frac{1}{I_h}\left|\iint_S P^* \, dxdy\right| \ll \sqrt{\iint_S I_h |P^*|^2 \, dxdy} \qquad (24)$$

with the same effect.

## Claims

1. A local hyperthermia ultrasonic irradiation system with annular focal zone comprising:

   a transducer (1) having a generally circular shape and being divided into a plurality of elements at least in a circumferential direction, the transducer (1) being adapted to generate a plurality of sound pressure regions on a focal plane;

   a drive circuit (8) for generating drive signals for driving each of the elements, respectively;

   characterised in that:

   the system has a control circuit (10,11) for controlling the phases of the drive signals such that the modulus or root mean square value of the integral over the focal plane of the sound pressures of the sound pressure regions is negligible compared with the integral of the modulus or root mean square value of the sound pressures over the focal plane.

2. A system according to claim 1, wherein the control circuit (10,11) is adapted to control the phases of the respective drive signals of the transducer elements such that the phases of the drive signals are rotatable through n rotations on the face of said transducer (1) in the circumferential direction.

3. A system according to claim 2, wherein the control circuit (10,11) is adapted to rotate the distribution of the respective drive phases of the oscillating elements while holding constant the mutual relationships between the drive phases.

4. A system according to any one of claims 1 to 3, wherein the transducer (1) includes geometric focusing means.

5. A system according to claim 4, wherein the geometric focusing means is an acoustic lens having a predetermined focal point.

6. A system according to claim 4, wherein the geometric focusing means is means for making the face of the transducer concave.

7. A system according to claim 1, further comprising:

   an array type monitoring probe at the centre of the face of the transducer (1); and

   imaging means for imaging by means of sound waves from an object by electronic linear or sector scanning of the array type probe.

8. A system according to claim 7, wherein the array type probe is driven by a signal having a frequency at least twice that of the drive signals for driving the transducer (1).

9. A local hyperthermia ultrasonic irradiation system with annular focal zone comprising:

   a transducer (1) having a generally circular contour and being divided into a plurality of elements in both circumferential and radial directions;

   a drive circuit (8) for driving each of the elements respectively;

   characterised in that:

   the system has a control circuit (10,11) for controlling the phases of drive signals for driving the elements, respectively, wherein the control circuit (10,11) is adapted to control the phases of the respective drive signals for driving the transducer elements such that sound waves may be converged in a desired annular focal zone and such that the modulus of the integral of the sound pressure over the focal plane is negligible compared with the integral of the modulus of the sound pressure over the same plane.

**10.** A system according to claim 9, wherein the control circuit (10,11) is adapted to determine the term o a phase adjustment corresponding to the radial positions o the elements with respect to the distance to, and the radius of the focal plane o the annular focal zone and is adapted to control the phases o the respective drive signals for driving the elements such that the phases o the drive signals are rotatable through n rotations in the circumferential direction o the face o the transducer (1).

**11.** A system according to claim 9, wherein the control circuit (10,11) is adapted to determine the term o a phase adjustment corresponding to the radial positions o the elements for converging sound waves at the centre position o the focal plane o the annular focal zone and is adapted to control the phases o the respective drive signals o the elements such that the phases o said drive signals are rotatable through n rotations in the circumferential direction o the face o the transducer (1).

**12.** A local hyperthermia ultrasonic irradiation system with annular focal zone comprising:
a transducer (1) having a plurality o elements arranged in a two-dimensional array;
a drive circuit (8) for driving the elements respectively;
characterised in that:
the system has a control circuit (10,11) for controlling the phases o drive signals for driving the elements, respectively, wherein the control circuit (10,11) is adapted to determine the term o a phase adjustment corresponding to the respective radial positions o transducer elements such that the integral o the sound pressure over the focal plane is negligible compared with the integral o the modulus o the sound pressure over the same plane, and such that sound waves are convergeable at the centre position o the focal plane o a desired annular focal zone, and is adapted to reverse sequentially the polarities o the drive signals in a predetermined direction o the two-dimensional array.

**13.** A local hyperthermia ultrasonic irradiation system with annular focal zone comprising:
a transducer (1) having a plurality o elements arranged in an annular array;
a drive circuit (8) for driving the elements respectively;
characterised in that:
the system has a control circuit (10,11) for controlling the phases o drive signals for driving said elements, respectively, wherein the control circuit controls the phases o the respective drive signals o the transducer elements such that the integral o the sound pressure over the focal plane is negligible compared with the integral o the modulus o the sound pressure over the same plane, and such that sound waves are convergeable toward an annular focal zone having a desired radius and a desired depthwise position.

**Revendications**

**1.** Système d'irradiation locale hyperthermique par ultrasons comprenant une zone focale annulaire comportant :
un transducteur (1) possédant une forme générale circulaire et subdivisé en une pluralité d'éléments au moins dans une direction circonférentielle, le transducteur (1) étant adapté pour produire une pluralité de zones de pression acoustique dans un plan focal;
un circuit d'excitation(8) pour produire des signaux d 'excitation pour exciter respectivement chacun des éléments;
caractérisé en ce que :
le système possède un circuit de commande (10,11) pour commander les phases des signaux d'excitation de manière que le module ou la valeur efcace de l'intégrale dans le plan focal des pressions acoustiques des zones de pression acoustique soient négligeables par rapport à l'intégrale du module ou de la valeur efcace des pressions acoustiques dans le plan focal.

**2.** Système selon la revendication 1, dans lequel le circuit de commande (10,11) est adapté pour commander les phases des signaux d'excitation respectis des éléments transducteurs de manière que les phases des signaux d'excitation puissent tourner sur n tours sur la face dudit transducteur (1) dans la direction circonférentielle.

**3.** Système selon la revendication 2, dans lequel le circuit de commande (10,11) est adapté pour faire tourner la distribution des phases respectives d'excitation des éléments oscillants, tout en maintenant constantes les relations mutuelles entre les phases d 'excitation.

**4.** Système selon l'une quelconque des revendications 1 à 3, dans lequel le transducteur (1) comprend des moyens géométriques de focalisation.

**5.** Système selon la revendication 4, dans lequel les moyens géométriques de focalisation sont constitués par une lentille acoustique possédant un foyer prédéterminé.

**6.** Système selon la revendication 4, dans lequel les moyens géométriques de focalisation sont des moyens permettant de rendre concave la face du transducteur.

**7.** Système selon la revendication 1, comportant en outre :

une sonde de contrôle du type en forme de réseau, placée au centre de la face du transducteur (1); et

des moyens de formation d'images pour former des images à l'aide d'ondes acoustiques provenant d'un objet, au moyen d'un balayage électronique linéaire ou sectoriel de la sonde du type en forme de réseau.

**8.** Système selon la revendication 7, dans lequel la sonde du type en forme de réseau est commandée par un signal possédant une féquence égale au moins au double de celle des signaux d'excitation pour la commande du transducteur (1).

**9.** Système d'irradiation locale hyperthermique à ultrasons comprenant une zone focale annulaire, comportant :

un transducteur (1) possédant un contour de forme générale circulaire et subdivisé en une pluralité d'éléments à la fois dans les directions circonférentielles et radiales;

un circuit d'excitation (8) pour exciter respectivement chacun des éléments;

caractérisé en ce que :

le système possède un circuit de commande (10,11) pour commander les phases de signaux d'excitation pour exciter respectivement les éléments, le circuit de commande (10,11) étant adapté pour commander les phases des signaux d 'excitation respectifs pour commander les éléments transducteurs de manière à pouvoir faire converger les ondes acoustiques en direction d'une zone focale annulaire désirée, et de manière que le module de l'intégrale de la pression acoustique dans le plan focal soit négligeable par rapport à l'intégrale du module de la pression acoustique dans le même plan.

**10.** Système selon la revendication 9, dans lequel le circuit de commande (10,11) est adapté pour déterminer le terme d'un ajustement de phase correspondant aux positions radiales des éléments en rapport avec la distance au plan focal de la zone focale annulaire et le rayon de ce plan et est adapté pour commander les phases des signaux d'excitation respectifs pour commander les éléments de manière que les phases des signaux d 'excitation puissent tourner sur n tours dans la direction circonférentielle de la face du transducteur (1).

**11.** Système selon la revendication 9, dans lequel le circuit de commande (10,11) est adapté pour déterminer le terme d'un ajustement de phase correspondant aux positions radiales des éléments pour faire converger des ondes acoustiques en direction de la position centrale du plan focal de la zone focale annulaire et est adapté pour commander les phases des signaux d 'excitation respectifs des éléments de manière que les phases desdits signaux de commande puissent tourner sur n tours dans la direction circonférentielle de la face du transducteur (1).

**12.** Système d'irradiation locale hyperthermique à ultrasons comprenant une zone focale annulaire, comportant :

un transducteur (1) possédant une pluralité d'éléments disposés selon un réseau bidimensionnel;

un circuit d'excitation (8) servant à exciter respectivement les éléments;

caractérisé en ce que :

le système possède un circuit de commande (10,11) pour commander les phases de signaux d'excitation pour respectivement commander les éléments, le circuit de commande (10,11) étant adapté pour déterminer le terme d'un ajustement de phase correspondant aux positions radiales respectives des éléments transducteurs de manière que l'intégrale de la pression acoustique dans le plan focal soit négligeable par rapport à l'intégrale du module de la pression acoustique dans le même plan, et de manière que les ondes acoustiques puissent converger en direction de la position centrale

EP 0 214 782 B1

du plan focal d'une zone focale annulaire désirée, et étant adapté pour inverser séquentiellement les polarités des signaux d 'excitation dans une direction prédéterminée du réseau bidimensionnel.

13. Système d'irradiation locale hyperthermique à ultrasons comprenant une zone focale annulaire, comportant :

un transducteur (1) possédant une pluralité d'éléments disposés suivant un réseau annulaire;
un circuit d 'excitation (8) pour exciter respectivement les éléments;
caractérisé en ce que :
le système possède un circuit de commande (10,11) pour commander les phases de signaux d'excitation pour respectivement exciter lesdits éléments, le circuit de commande commandant les phases des signaux d'excitation respectifs des éléments transducteurs de manière que l'intégrale de la pression acoustique dans le plan focal soit négligeable par rapport à l'intégrale du module de la pression acoustique dans le même plan, et de manière que des ondes acoustiques puissent converger en direction d'une zone focale annulaire possédant un rayon désiré et une position désirée en profondeur.

## Patentansprüche

1. Ultraschall-Bestrahlungsanordnung zur lokalen Übererwärmung mit ringförmiger Brennpunktszone und mit

einem Wandler (1) von im allgemeinen runder Form, der wenigstens in Umfangsrichtung in eine Anzahl von Elementen aufgeteilt ist, wobei der Wandler (1) dafür vorgesehen ist, in einer Brennebene eine Anzahl von Schalldruckbereichen zu erzeugen;
einer Ansteuerschaltung (8) zum Erzeugen von Ansteuersignalen zum Ansteuern jedes der Elemente;
dadurch gekennzeichnet, daß
die Anordnung eine Steuerschaltung (10, 11) zum Steuern der Phasen der Ansteuersignale derart aufweist, daß der Absolutbetrag oder quadratische Mittelwert des Integrals über die Brennebene der Schalldrücke der Schalldruckbereiche im Vergleich zum Integral des Absolutbetrages oder quadratischen Mittelwertes der Schalldrücke über die Brennebene vernachlässigbar ist.

2. Anordnung nach Anspruch 1, wobei die Steuerschaltung (10, 11) dafür vorgesehen ist, die Phasen der jeweiligen Ansteuersignale der Wandlerelemente derart zu steuern, daß die Phasen der Ansteuersignale über n Drehungen an der Vorderseite des Wandlers (1) in Umfangsrichtung drehbar sind.

3. Anordnung nach Anspruch 2, wobei die Steuerschaltung (10, 11) dafür vorgesehen ist, die Verteilung der jeweiligen Ansteuerphasen der Schwingelemente zu drehen, während die gegenseitigen Beziehungen zwischen den Ansteuerphasen konstant gehalten werden.

4. Anordnung nach einem der Ansprüche 1 bis 3, wobei der Wandler (1) eine geometrische Fokussiereinrichtung aufweist.

5. Anordnung nach Anspruch 4, wobei die geometrische Fokussiereinrichtung eine akustische Linse mit vorgegebenem Brennpunkt ist.

6. Anordnung nach Anspruch 4, wobei die geometrische Fokussiereinrichtung eine Einrichtung ist, die die Vorderseite des Wandlers konkav macht.

7. Anordnung nach Anspruch 1, mit

einem Kontroll-Prüfkop des Arraytyps in der Mitte der Vorderseite des Wandlers (1); und mit einer Abbildungseinrichtung zur Erzeugung einer Abbildung mittels Schallwellen von einem Objekt durch elektronische lineare oder Sektor-Abtastung des Prüfkops vom Arraytyp.

8. Anordnung nach Anspruch 7, wobei der Prüfkop des Arraytyps durch ein Signal angesteuert wird, das eine Frequenz von wenigstens dem Doppelten der Frequenz der Ansteuersignale zum Ansteuern des Wandlers (1) hat.

9. Ultraschall-Bestrahlungsanordnung zur lokalen Übererwärmung mit ringförmiger Brennpunktszone und

13

mit

einem Wandler (1) von im allgemeinen runder Form, der sowohl in Umangsrichtung als auch in radialer Richtung in eine Anzahl von Elementen augeteilt ist;

einer Ansteuerschaltung (8) zum Ansteuern jedes der Elemente;

dadurch gekennzeichnet, daß

die Anordnung eine Steuerschaltung (10, 11) zum Steuern der Phasen der Ansteuersignale zum Ansteuern der Elemente aufweist, wobei die Steuerschaltung (10, 11) dafür vorgesehen ist, die Phasen der jeweiligen Ansteuersignale zum Ansteuern der Wandlerelemente derart zu steuern, daß die Schallwellen in einer erwünschten ringförmigen Brennpunktszone konvergieren und daß der Absolutbetrag des Integrals des Schalldruckes über die Brennebene im Vergleich zum Integral des Absolutbetrages des Schalldruckes über die gleiche Ebene vernachlässigbar ist.

**10.** Anordnung nach Anspruch 9, wobei die Steuerschaltung (10, 11) dafür vorgesehen ist, den Term einer Phaseneinstellung entsprechend der radialen Positionen der Elemente bezüglich des Abstandes zu, und dem Radius von, der Brennebene der ringförmigen Brennpunktszone zu bestimmen und dafür vorgesehen ist, die Phasen der jeweiligen Ansteuersignale zum Ansteuern der Elemente derart zu steuern, daß die Phasen der Ansteuersignale über n Drehungen in der Umangsrichtung der Vorderseite des Wandlers (1) drehbar sind.

**11.** Anordnung nach Anspruch 9, wobei die Steuerschaltung (10, 11) dafür vorgesehen ist, den Term einer Phaseneinstellung entsprechend der radialen Positionen der Elemente zum Konvergieren der Schallwellen an der Position des Mittelpunktes der Brennebene der ringförmigen Brennpunktszone zu bestimmen und dafür vorgesehen ist, die Phasen der jeweiligen Ansteuersignale für die Elemente derart zu steuern, daß die Phasen der Ansteuersignale über n Drehungen in der Umangsrichtung der Vorderseite des Wandlers (1) drehbar sind.

**12.** Ultraschall-Bestrahlungsanordnung zur lokalen Übererwärmung mit ringförmiger Brennpunktszone und mit

einem Wandler (1) mit einer Anzahl von Elementen, die in einem zweidimensionalen Array angeordnet sind;

einer Ansteuerschaltung (8) zum Ansteuern jedes der Elemente;

dadurch gekennzeichnet, daß

die Anordnung eine Steuerschaltung (10, 11) zum Steuern der Phasen der Ansteuersignale zum Ansteuern der Elemente aufweist, wobei die Steuerschaltung (10, 11) dafür vorgesehen ist, den Term einer Phaseneinstellung entsprechend den radialen Positionen der Wandlerelemente derart zu bestimmen, daß das Integral des Schalldruckes über die Brennebene im Vergleich zum Integral des Absolutbetrages des Schalldruckes über die gleiche Ebene vernachlässigbar ist, und derart, daß die Schallwellen an der Position des Mittelpunkts der Brennebene einer erwünschten ringförmigen Brennpunktszone konvergierbar sind, und daß sie dafür vorgesehen ist, aufeinanderfolgend die Polaritäten der Ansteuersignale in einer vorgegebenen Richtung des zweidimensionalen Arrays umzukehren.

**13.** Ultraschall-Bestrahlungsanordnung zur lokalen Übererwärmung mit ringförmiger Brennpunktszone und mit

einem Wandler (1) mit einer Anzahl von Elementen, die in einem ringförmigen Array angeordnet sind;

einer Ansteuerschaltung (8) zum Ansteuern jedes der Elemente;

dadurch gekennzeichnet, daß

die Anordnung eine Steuerschaltung (10, 11) zum Steuern der Phasen der Ansteuersignale zum Ansteuern der Elemente aufweist, wobei die Steuerschaltung die Phasen der jeweiligen Ansteuersignale der Wandlerelemente derart steuert, daß das Integral des Schalldruckes über die Brennebene im Vergleich zum Integral des Absolutbetrages des Schalldruckes über die gleiche Ebene vernachlässigbar ist, und daß die Schallwellen zu einer ringförmigen Brennpunktszone mit einem erwünschten Radius und einer erwünschten Tiefen-Position konvergierbar sind.

# FIG. 1 PRIOR ART

# FIG. 2

# FIG. 5

## FIG. 3

## FIG. 4A

## FIG. 4B

## FIG. 6A

## FIG. 6B

FIG. 7

FIG. 8A

FIG. 8B

7

6

1

2

3

4

FIG. 9A

FIG. 9B

FIG. 10A

FIG. 10B

FIG. 10C

FIG. 11A

FIG. 11B

FIG. 11C

*FIG. 12A*

*FIG. 12B*

24

# FIG. 13A

n = 4

Z = 80mm = $Z_F$

# FIG. 13B

n = 8

Z = 80mm = $Z_F$

FIG. 14A

FIG. 14B